(19) Europäisches Patentamt / European Patent Office / Office européen des brevets

(11) **EP 4 431 103 A1**

(12) **EUROPEAN PATENT APPLICATION**

(43) Date of publication:
**18.09.2024   Bulletin 2024/38**

(21) Application number: **23162026.1**

(22) Date of filing: **15.03.2023**

(51) International Patent Classification (IPC):
**A61K 31/727** (2006.01)      **A61K 9/20** (2006.01)
**A61P 31/12** (2006.01)

(52) Cooperative Patent Classification (CPC):
**A61K 31/727; A61K 9/2027; A61K 9/2054;
A61P 31/12**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Zona Swiss SA
6537 Grono (CH)**

(72) Inventors:
- **RONCHI, Federica
  20124 MILANO (IT)**
- **RONCHI, Celestino
  20124 MILANO (IT)**

(74) Representative: **Bianchetti & Minoja with
Trevisan & Cuonzo IPS SRL
Via Plinio, 63
20129 Milano (IT)**

(54) **FORMULATIONS COMPRISING HEPARAN SULFATE FOR THE TREATMENT AND
PREVENTION OF VIRAL INFECTIONS CAUSED BY ORTHOCORONAVIRIDAE**

(57)    The present invention relates to inhalation formulations comprising heparan sulfate as active ingredient and a suitable excipient, which are useful for the treatment and prevention of viral infections caused by Orthocoronavirinae.

**EP 4 431 103 A1**

**Description**

The invention relates to formulations comprising heparan sulfate as active ingredient and a suitable carrier, which are useful for the treatment and prevention of viral infections caused by Orthocoronaviridae.

**Prior art**

Orthocoronavirinae is a subfamily of viruses, also known as coronaviruses, belonging to the Coronaviridae family, suborder Comidovirineae, order Nidovirales.

It is divided into the genera Alphacoronavirus, Betacoronavirus, Gammacoronavirus and Deltacoronavirus, which include phylogenetically compact positive-sense RNA genogroups having a nucleocapsid with helical symmetry. Alphacoronaviruses and betacoronaviruses derive from the bat gene pool.

The genomic size of coronaviruses ranges between about 26 and 32 kilobases, which is exceptionally large for an RNA virus.

Their number is growing rapidly, and several new coronaviruses have been discovered recently, including SARS-CoV-1 in 2002, MERS-CoV in 2012 and SARS-CoV-2 in Wuhan (China) in 2019. Coronaviruses are responsible for a number of diseases affecting mammals and birds.

In humans they cause respiratory tract infections, which are often mild, like the common cold, but in rare cases are potentially lethal, such as pneumonia and bronchitis. Coronaviruses are responsible for serious epidemics of SARS in November 2002, MERS in 2012 and the new COVID-19 pandemic.

It is known that protein ACE2, a type I integral membrane protein abundantly expressed in the tissues lining the respiratory tract, is necessary for viral entry but is not the primary binding site on the cell surface. It has been demonstrated (Milewska A. et al., J Virol. 2014 Nov; 88(22): 13221-30. Epub 2014 Sep.3) that heparan sulfate proteoglycans act as adhesion molecules for coronavirus, increasing the density of the virus on the cell surface and possibly facilitating the interaction between HCoV and its receptor (ACE2).

Heparan sulfate (HS) is a sulphurylated glycosaminoglycan (GAG) present on the cell surface and in the extracellular matrix of multicellular organisms.

HS has an average molecular weight of 50 KDa and consists of disaccharide units of alpha-D-glucosamine bound via bond 1-4 to a unit of uronic acid. Heparan sulfate, in form of tablets for oral administration, is used for the treatment of vascular pathologies with thrombotic risk. Heparan sulfate is also used topically for the treatment of phlebitis and, in general, of cutaneous conditions of vascular origin.

Donalisio M. et al. (ASM Journals, Antimicrobial Agents and Chemotherapy Vol.56, No. 10) highlight the interaction of respiratory syncytial virus (RSV) with heparan sulfate proteoglycans (HSPGs) on the cell surface to trigger infection. The virus-heparan sulfate interaction can therefore represent a target for the development of respiratory syncytial virus infection inhibitors. The study analysed a mini-library of linear, dimeric and dendrimeric peptides, containing clusters of basic amino acids, with a view to identifying peptides able to bind HSPGs, and therefore inhibit RSV adherence and infectivity.

Human metapneumovirus (HMPV) is a respiratory pathogen that causes acute diseases of the upper and lower respiratory tract. The mechanism whereby said virus recognises and accesses its target cell is still largely unknown. Chang A. et al. (ASM Journals - Journal of Virology, Vol.86, No.6) studied the initial stages of the bond with the virus and the consequent infection and discovered that the first binding partner for HMPV is HS.

Clausen et al. (Cell 183, 1043-1057, 2020) have demonstrated that heparan sulfate is a cofactor which is also necessary for SARS-CoV-2 infection; heparan sulfate interacts with the domain of the bond to the SARS-CoV-2 spike glycoprotein receptor, modifying its structure to facilitate its binding to ACE2. The authors suggest that HS mimetics, HS-degrading lyases and HS biosynthesis inhibitors could be used for therapeutic purposes.

EP 1 513 502 discloses inhalation formulations comprising glycosaminoglycans as adjuvants for the release of therapeutic agents such as bronchodilators, steroids, antibacterials and antivirals.

WO 2021/194890 discloses associations of heparin and N-acetylcysteine, administered by inhalation, which are useful for the treatment of respiratory infections caused by coronaviruses, optionally in combination with antivirals such as remdesivir, tocilizumab, lopinavir, sarilumab and beta interferon. None of the prior art documents therefore describe or postulate direct therapeutic applications of unmodified heparan sulfate as an agent able to treat or prevent viral infections.

WO 2021/211243 discloses dextran sulfate for use in the treatment of a coronavirus infection.

**Description of the invention**

It has now been discovered that heparan sulfate, when administered either systemically, effectively counteracts SARS-CoV2 infection by occupying as antagonist the binding site of the virus to the proteoglycans present on the cell membranes and extracellular matrices.

Heparan sulfate acts as an adherence molecule which occupies the binding site of the virus to the cells.

The invention provides therefore formulations comprising heparan sulfate (HS) as active ingredient and suitable excipients According to the invention, heparan sulfate may be administered by the oral or parenteral route.

Examples of formulations suited for the oral administration include tablets, immediate release tablets, controlled release tablets, capsules, pills, syrups, granules and the like. Conventional excipients may be used, such as diluents, binders, granulating agents, lubricants.

Suitable excipients for immediate release tablets comprise lactose, microcrystalline cellulose, povidone, magnesium stearate, talc.

Suitable excipients for controlled release tablets comprise lactose, microcrystalline cellulose, povidone, magnesium stearate, talc, HPMC, carbomer.

Suitable excipients for oral capsules (gelatin or HPMC capsules) comprise lactose, microcrystalline cellulose, povidone, magnesium stearate, talc.

Suitable excipients for granules comprise lactose, mannitol xylitol, sweeteners, flavorings and coloring agents.

Suitable excipients for oral solutions comprise buffers, water, hydro soluble excipients, preservatives and antioxidants.

Heparan sulfate can be administered orally at doses ranging from 10 to 500 mg, once or twice a day.

For the treatment and prevention of SARS CoV2 infections, the formulations according to the invention can be administered one to three times a day, depending on the clinical response, the severity of the disease and the patient's condition.

The effectiveness of heparan sulfate as antiviral agent against SARS-CoV-2 has been studied in viral titration experiments on Vero E6 cells, as reported below.

The SARS-CoV-2 ancestral strain was used to infect Vero E6 cells and evaluate the antiviral activity of heparan sulfate at the concentration of 8 $\mu$g/ml under 4 different conditions:

1) Vero E6 cells pre-exposed to heparan sulfate and then infected by the virus (CHS+V);
2) Vero E6 cells infected with heparan sulfate pre-exposed virus (C+VHS);
3) Vero E6 cells infected with the virus and subsequently exposed to heparan sulfate (C+V+HS);
4) Vero E6 cells infected with virus (C+V).

These 4 experimental conditions were evaluated for different exposure times: 24 hours, 48 hours, 72 hours, administration of repeated doses every 24 hours (for a total exposure of 72 hours).

Viral pre-exposure (C+VHS) was obtained by incubating the virus for 2 hours at 37°C and 5% $CO_2$. The pre-exposed virus was then used to infect the cells (2 hours at 37°C and 5% $CO_2$). After removing the inoculum, heparan sulfate was added and remained in contact with the infected cells for the various exposure times (24h, 48h, 72h).

For exposure of infected cells to heparan sulfate (C+V+HS), infection was performed with unexposed virus and heparan sulfate was added after removal of the viral inoculum and maintained for the different exposure times (24h, 48h, 72h).

72 hours after infection, the supernatant (containing the virus) was titrated to determine the viral concentration under the different experimental conditions and thus allow a quantitative assessment of the antiviral activity of heparan sulfate.

For each condition, 4 replicates were titrated using the Reed and Muench method:

$$PD = \frac{\% \, of \, dilution \, above \, 50\% - 50}{\% \, of \, dilution \, above \, 50\% - \% \, of \, dilution \, below \, 50\%}$$

The statistical analysis was carried out by applying the Student's T test with Welch's correction on the log10 values of the viral titer.

**Results**

The viral titration results for each condition with 24 hours exposure (Table 1), 48 hours exposure (Table 2), 72 hours exposure (Table 3), and repeated doses (Table 4), are shown below.

Table 1. Results of viral titration after 24 h exposure.

| Exposure time: 24 hours | | | |
|---|---|---|---|
| Condition | Mean of viral titer (TCID50/ml) | Mean of viral titer ($Log_{10}$ TCID50/ml) | Percentage of viral reduction compared with control (C+V) |
| CHS + V | $9 \times 10^7$ | 7.25 | 0% |
| C + VHS | $3.6 \times 10^5$ | 5.40 | 25.5% |
| C + V + HS | $9 \times 10^6$ | 6.30 | 13% |
| C + V | $9 \times 10^7$ | 7.25 | |

Table 2. Results of viral titration after 48 h exposure.

| Exposure time: 48 hours | | | |
|---|---|---|---|
| Condition | Mean of viral titer (TCID50/ml) | Mean of viral titer ($Log_{10}$ TCID50/ml) | Percentage of viral reduction compared with control (C+V) |
| CHS + V | $1.69 \times 10^8$ | 7.80 | 0% |
| C + VHS | $9.7 \times 10^5$ | 5.50 | 29.5% |
| C + V + HS | $1 \times 10^7$ | 6.65 | 14.7% |
| C + V | $1.69 \times 10^8$ | 7.80 | |

Table 3. Results of viral titration after 72 h exposure.

| Exposure time: 72 hours | | | |
|---|---|---|---|
| Condition | Mean of viral titer (TCID50/ml) | Mean of viral titer ($Log_{10}$ TCID50/ml) | Percentage of viral reduction compared with control (C+V) |
| CHS + V | $3.64 \times 10^8$ | 8.45 | 0% |
| C + VHS | $2.9 \times 10^5$ | 5.35 | 37.7% |
| C + V + HS | $2.9 \times 10^6$ | 6.3 | 25.4% |
| C + V | $3.64 \times 10^8$ | 8.45 | |

Table 4. Results of viral titration after repeated exposures.

| Repeated Doses | | | |
|---|---|---|---|
| Condition | Mean of viral titer (TCID50/ml) | Mean of viral titer ($Log_{10}$ TCID50/ml) | Percentage of viral reduction compared with control (C+V) |
| CHS + V | $2.48 \times 10^8$ | 8.25 | 0% |
| C + VHS | $2.9 \times 10^5$ | 5.35 | 35% |
| C + V + HS | $2.9 \times 10^6$ | 6.3 | 23.6% |
| C + V | $2.48 \times 10^8$ | 8.25 | |

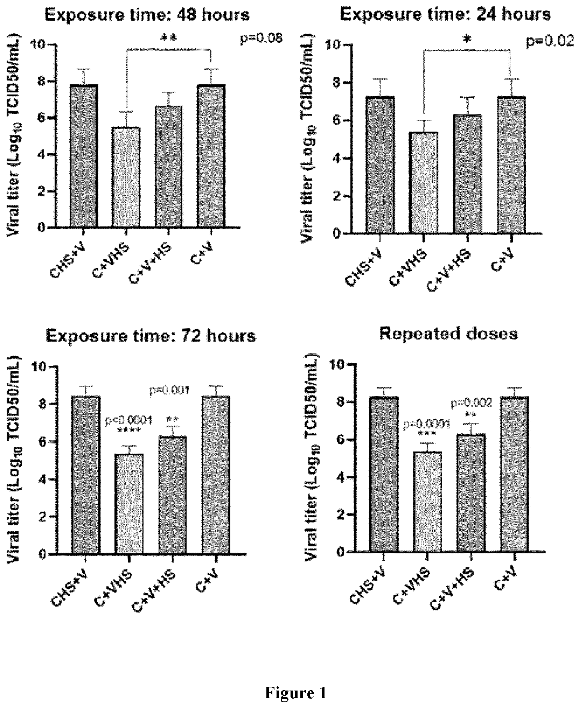

The greatest antiviral activity was observed by pre-treatment of the virus with heparan sulfate (pre-exposure of virus + heparan sulfate for 2 hours and subsequent addition of heparan sulfate after removal of the inoculum for 24/48/72 hours). This result is in agreement with the activity of heparan sulfate which probably binds to viral receptors during pre-exposure and thus prevents the binding of viral particles to cells, reducing infection. For this condition, a statistically significant reduction of the viral titer was found compared to the control for all 4 exposure times: 24h (p=0.02), 48h (p=0.08), 72h (p<0.0001), repeated doses (p=0.0001) (Figure 1).

However, the antiviral activity due to the administration of heparan sulfate on infected cells (i.e. after removal of the viral inoculum) was confirmed. The absence of viral pre-exposure reduces the effectiveness of infection reduction but still maintains a good antiviral effect. Probably, the heparan sulfate added to infected cells still manages to bind the receptors of the new viral particles, thus preventing their binding with new cells and thus slowing down the infection. For this experimental condition, a statistically significant reduction in the viral titer was found compared to the positive control for the exposure times of 72h (p=0.001) and repeated doses (p=0.002) (Figure 1).

As regards the exposure time, the greater efficacy of a 72-hour exposure has been confirmed.

Detailed examples of formulations according to the invention are set out below.

**Example 1 immediate release tablet containing 100.0 mg of heparan sulfate**

Qualitative/quantitative composition for a tablet containing 100.0 mg of HS

| | | |
|---|---|---|
| ○ | HS | 100,0 mg |
| ○ | Lactose | 93, 0 mg |
| ○ | Microcrystalline cellulose | 200,0 mg |
| ○ | Talc | 5,0 mg |
| ○ | Magnesium Stearate | 2,0 mg |
| Total weight | | 400,0 mg |

**Example 2: slow release tablet containing 150.0 mg of heparan sulfate**

Qualitative/quantitative composition for a slow release tablet containing 150.0 mg of HS

| | | |
|---|---|---|
| ○ | HS | 150,0 mg |
| ○ | Mannitol | 113,0 mg |
| ○ | Polivinyl pyrrolidone | 10,0 mg |
| ○ | Methyl hydroxy cellulose HV | 100,0 mg |
| ○ | Talc | 5,0 mg |
| ○ | Glyceryl Behenate | 2,0 mg |
| *Total weight* | | *380,0 mg* |

**Claims**

1. Formulations comprising heparan sulfate for use in the treatment and prevention of infections caused by Orthocoronaviridae.

2. Formulations for use according to claim 1 wherein the infections are caused by SARS CoV2.

3. Formulations for use according to claim 1 or 2 for oral or parenteral administration.

4. Formulations for use according to any one of claims 1-3 in form of immediate release tablets, controlled release tablets, oral capsules, granules, oral solutions,

5. Formulations for use according to claim 4 in the form of immediate release tablets.

6. Formulations for use according to claim 4 in the form of controlled release tablets.

7. Formulations for use according to claim 4 in the form of oral capsules.

8. Formulations for use according to claim 4 in the form of granules.

9. Formulations for use according to claim 4 in the form of oral solutions.

10. Formulations for use according to any one of claims 1 to 9 comprising 5 to 500 mg of heparan sulfate per dosage unit.

**Figure 1**

EUROPEAN SEARCH REPORT

Application Number

EP 23 16 2026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/194890 A1 (ATOSSA THERAPEUTICS INC [US]) 30 September 2021 (2021-09-30) | 1-10 | INV. A61K31/727 A61K9/20 A61P31/12 |
| Y | * claims 37, 41, 65 *<br>* page 1, paragraph 3 *<br>* page 57, paragraph 165 *<br>----- | 3-9 | |
| X | WO 2021/215987 A1 (BONOSS MEDICAL AB [SE]) 28 October 2021 (2021-10-28) | 1,2,10 | |
| Y | * claims 1, 6, 13 *<br>* page 11, lines 5-10 *<br>* page 12, lines 8-11 *<br>* page 20, lines 22-29 *<br>----- | 3-9 | |
| E | EP 4 197 524 A1 (CGM RES S A S DI MARCO ROSSI [IT]) 21 June 2023 (2023-06-21)<br>* claims 1, 6 *<br>* page 2, paragraph 1 *<br>* page 2, paragraph 17-19 *<br>----- | 1,2,10 | |
| X | GUIMOND SCOTT E. ET AL: "Synthetic Heparan Sulfate Mimetic Pixatimod (PG545) Potently Inhibits SARS-CoV-2 by Disrupting the Spike-ACE2 Interaction",<br>ACS CENTRAL SCIENCE,<br>vol. 8, no. 5, 29 March 2022 (2022-03-29), pages 527-545, XP093074475,<br>ISSN: 2374-7943, DOI: 10.1021/acscentsci.1c01293<br>Retrieved from the Internet:<br>URL:https://pubs.acs.org/doi/pdf/10.1021/acscentsci.1c01293><br>* abstract *<br>----- | 1 | TECHNICAL FIELDS SEARCHED (IPC)<br><br>A61K<br>A61P |
| Y | US 2001/024658 A1 (CHEN FENG-JING [US] ET AL) 27 September 2001 (2001-09-27)<br>* claims 68, 76 *<br>-----<br>-/-- | 3-9 | |

The present search report has been drawn up for all claims

| Place of search | Date of completion of the search | Examiner |
|---|---|---|
| The Hague | 18 August 2023 | Collins, Sally |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 23 16 2026**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 95/12403 A1 (PHARMACIA AB [SE]; HANSON LARS AA [SE] ET AL.) 11 May 1995 (1995-05-11) * page 1, lines 5-16 * * page 5, lines 1-21 * ----- | 3-9 | |

TECHNICAL FIELDS
SEARCHED      (IPC)

The present search report has been drawn up for all claims

| Place of search | Date of completion of the search | Examiner |
|---|---|---|
| The Hague | 18 August 2023 | Collins, Sally |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**page 2 of 2**

**EP 4 431 103 A1**

## ANNEX TO THE EUROPEAN SEARCH REPORT
## ON EUROPEAN PATENT APPLICATION NO.

EP 23 16 2026

18-08-2023

| Patent document cited in search report | | Publication date | Patent family member(s) | | Publication date |
|---|---|---|---|---|---|
| WO 2021194890 | A1 | 30-09-2021 | AU | 2021241450 A1 | 15-09-2022 |
| | | | CA | 3169898 A1 | 30-09-2021 |
| | | | EP | 4125936 A1 | 08-02-2023 |
| | | | US | 2023110614 A1 | 13-04-2023 |
| | | | WO | 2021194890 A1 | 30-09-2021 |
| WO 2021215987 | A1 | 28-10-2021 | NONE | | |
| EP 4197524 | A1 | 21-06-2023 | NONE | | |
| US 2001024658 | A1 | 27-09-2001 | AU | 2002243387 A1 | 16-07-2002 |
| | | | US | 2001024658 A1 | 27-09-2001 |
| | | | WO | 02053100 A2 | 11-07-2002 |
| WO 9512403 | A1 | 11-05-1995 | AU | 8119594 A | 23-05-1995 |
| | | | WO | 9512403 A1 | 11-05-1995 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1513502 A
- WO 2021194890 A
- WO 2021211243 A

**Non-patent literature cited in the description**

- **MILEWSKA A. et al.** *J Virol.,* 03 September 2014, vol. 88 (22), 13221-30
- **DONALISIO M. et al.** *ASM Journals, Antimicrobial Agents and Chemotherapy,* vol. 56 (10
- **CHANG A. et al.** *ASM Journals - Journal of Virology,* vol. 86 (6
- **CLAUSEN et al.** *Cell,* 2020, vol. 183, 1043-1057